# EUROPEAN PATENT APPLICATION

(11) **EP 4 644 901 A2**
(43) Date of publication of application: **05.11.2025**
(21) Application number: 25168008.8
(22) Date of filing: 02.04.2025
(51) Int. Cl.: G01N 33/574

(54) **CHROMOGENIC MULTIPLEXING METHODS AND SYSTEMS FOR IDENTIFYING A CANCER OF UNKNOWN PRIMARY ORIGIN**

(30) Priority: 15.04.2024 US 202463633889 P
(71) Applicant: Leica Biosystems Newcastle Limited, Newcastle Upon Tyne NE12 8EW (GB)
(72) Inventor: FULLER, Andrew, Newcastle upon Tyne, NE12 8EW (GB); FERBER, David, Newcastle upon Tyne, NE12 8EW (GB)
(74) Representative: Simmons & Simmons

(57) **Abstract**

A method and apparatus for labeling a tissue section is provided. In certain aspects, the methods comprise labeling a tissue sample via a plurality of immunohistochemistry (IHC) assays for detection of markers for characterization of a cancer origin in an individual having a cancer of unknown primary (CUP). The disclosed IHC assays employ chromogen-based detection methods for improved sample efficiency and visualization of biomarkers. Further disclosed is an apparatus for carrying out the disclosed methods.

## Description

### CROSS-REFERENCE TO RELATED APPLICATIONS

The present application claims priority to U.S. Provisional Patent Application No. 63/633,889 entitled "Chromogenic Multiplexing Methods and Systems for Identifying a Cancer of Unknown Primary Origin," filed on April 15, 2024, the disclosure of which is incorporated by reference herein.

### BACKGROUND

While most patients with cancer present with a clearly defined primary tumor, about 10-15% of patients present initially with metastatic disease. In many of these patients, the site of origin will not be obvious and in about 1/3 of these cases the primary tumor may never be found. Thus, cancer of unknown primary ("CUP") is a common problem, representing one of the ten most common cancer diagnoses. (e.g. Schofield and Oien, (2018). Standards and datasets for reporting cancers; Dataset for histopathological reporting of cancer of unknown primary (CUP) and malignancy of unknown primary origin (MUO), The Royal College of Pathologists.) Identifying the type of cancer in cases where the origin is unknown remains a significant challenge in oncology. Exclusion or diagnosis of CUP/MUO is stepwise, using clinical context, morphology, immunohistochemistry, and molecular analysis. Immunohistochemistry (IHC), a technique which employs the staining of tissue samples for expression of certain markers, is a technique used to determine the origin of a CUP. IHC utilizes antibody-based detection of biological markers in a tissue sample. By comparing the protein expression pattern observed in a tissue to known patterns of various cancer types, the data obtained from an IHC assay, or multiple IHC assays, can be used for diagnosis, disease characterization, and/or for developing a personalized treatment plan.

While IHC methods to detect biomarkers are increasingly useful for the diagnosis and treatment of patients with a CUP, the increase in biomarkers being required for diagnosis and targeted care has led to an increasing amount of tissue being required for basic tumor characterization. Simultaneously, with advances in radiology and supporting technologies, biopsy samples are becoming smaller, making the availability of tissue samples limited. As such, there is an increased need for tissue samples and a simultaneous decrease in the amount of tissue sample available for such testing. Multiplex methods, in which more than one detectable agent (*e.g.,* an antibody specific for a biological marker) is applied to a single sample, allows for detection of more than one biomarker in a single tissue sample and therefore has the benefit of reducing the amount of tissue sample needed. However, even with the efficiency of multiplex IHC, there is a need for improved methods which provide useful diagnostic information with fewer biomarkers, such that limited tissue sample may be preserved and repeat biopsies may be avoided. The instant disclosure seeks to address one or more of the aforementioned needs in the art.

### BRIEF DESCRIPTION OF THE DRAWINGS

This application file contains at least one drawing executed in color. Copies of this patent or patent application publication with color drawing(s) will be provided by the Office upon request and payment of the necessary fee.

While the specification concludes with claims which particularly point out and distinctly claim this technology, it is believed this technology will be better understood from the following description of certain examples taken in conjunction with the accompanying drawings, in which like reference numerals identify the same elements and in which:

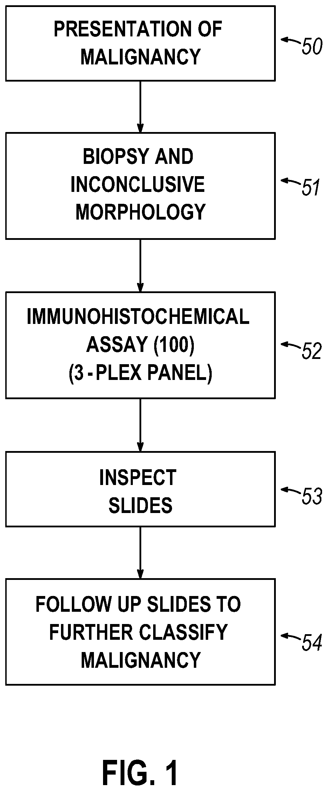

FIG. 1 depicts a flow chart of the process for determining the origin of a cancer of unknown primary (CUP) or unknown malignant lesion.

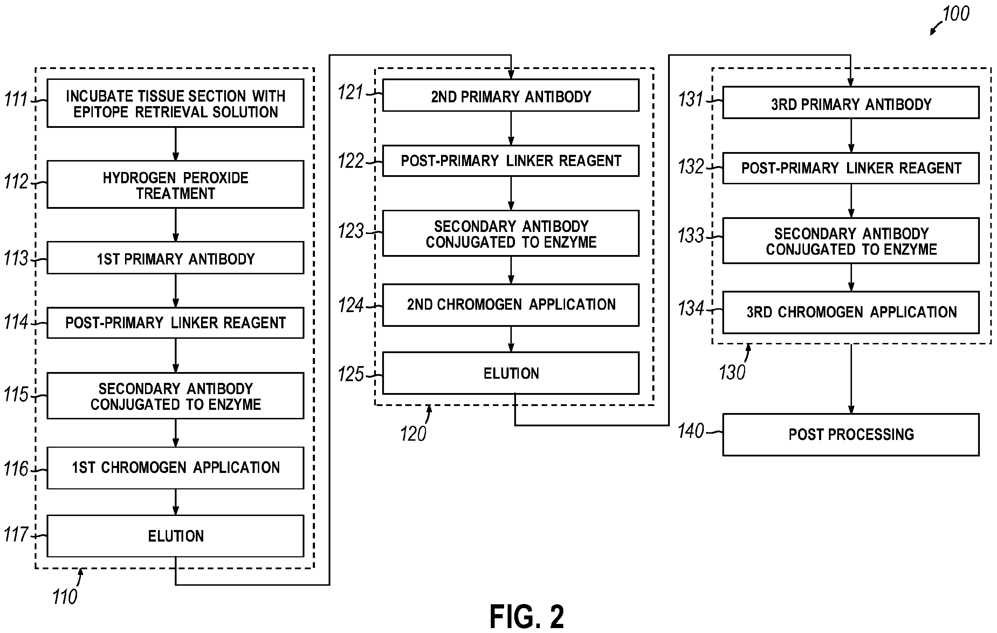

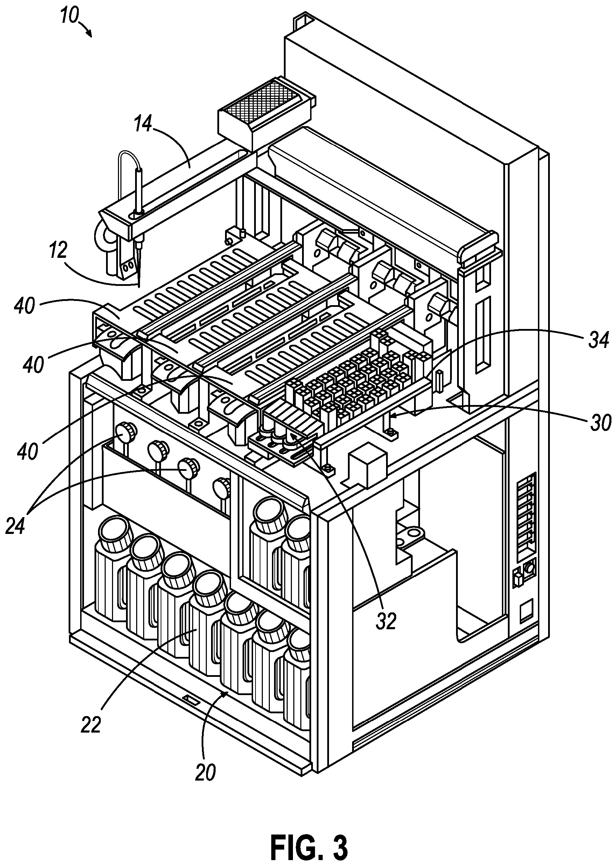

FIG. 2 depicts a flow chart of an exemplary triplex Immunohistochemistry (IHC) Assay Protocol (100) for use with a slide-staining instrument (10) of FIG. 3;
FIG. 3 depicts a perspective view of an example of a slide-staining instrument;

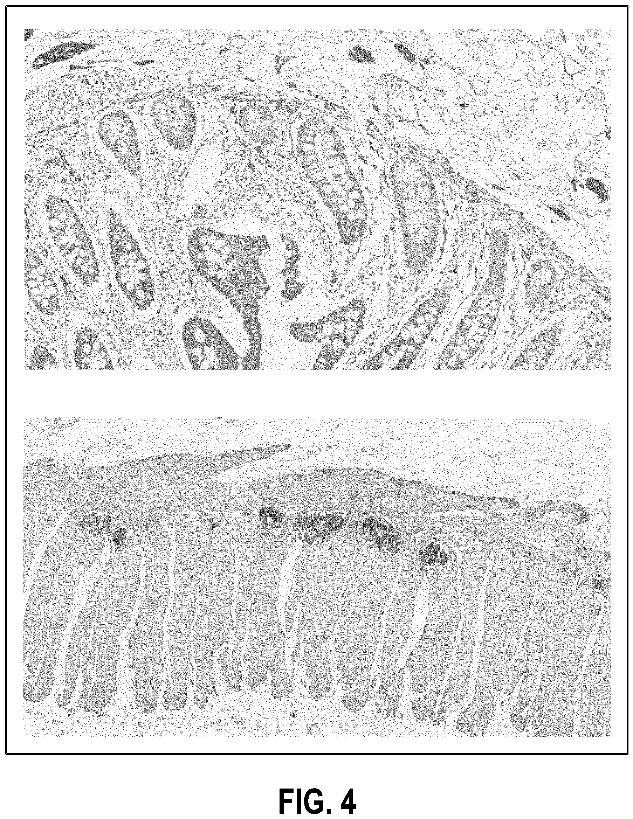

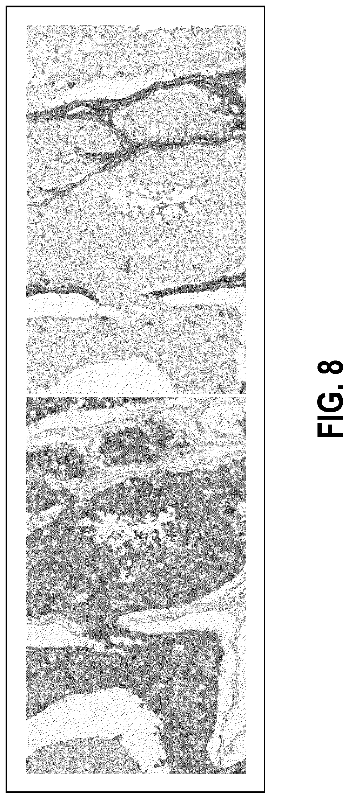

FIG. 4 (top panel) depicts an exemplary result of a triplex IHC panel using the disclosed methods on a tissue sample on a normal colon tissue sample. S100 is visualized via a blue chromogen, AE1/3 is visualized via a red chromogen, and desmin is visualized via a green chromogen. The bottom panel of FIG. 8 depicts a lower magnification of the muscle layer within this same tissue, illustrating that the desmin staining is detecting the correct cells

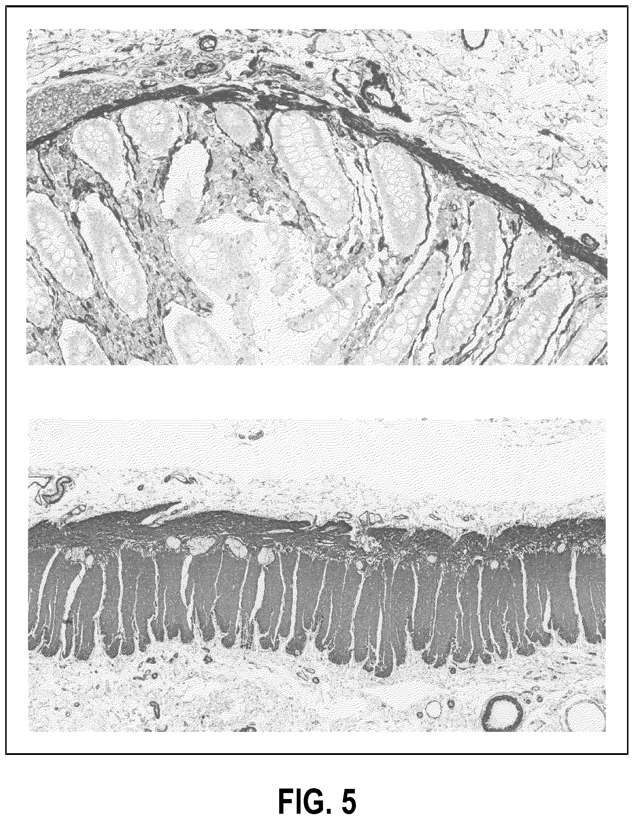

FIG. 5 (top panel) depicts an exemplary result of a triplex IHC panel using the disclosed methods on a tissue sample of normal colon tissue sample. SMA is visualized via a blue chromogen, CD34 is visualized via a red chromogen, and CD45 is visualized via a green chromogen. The bottom panel of FIG. 9 depicts a lower magnification of the same tissue showing the SMA staining of the muscle cells.

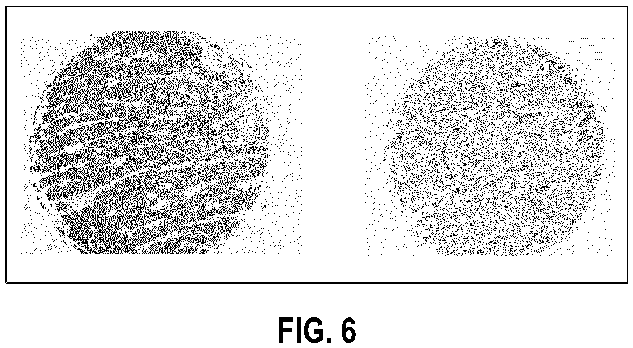

FIG. 6 depicts the results of two triplex IHC assays in which a tissue is assayed for all six markers described herein. On the left is a first triplex assay (a first panel) performed on a melanoma positive tissue sample. On the first panel, S100 is visualized via a blue chromogen, AE1/3 is visualized via a red chromogen, and desmin is visualized via a green chromogen. On the right is the results of a second triplex assay (a second panel), performed on the same melanoma positive tissue sample. On the second panel, SMA is visualized via a blue chromogen, CD34 is visualized via a red chromogen, and CD45 is visualized via a green chromogen.

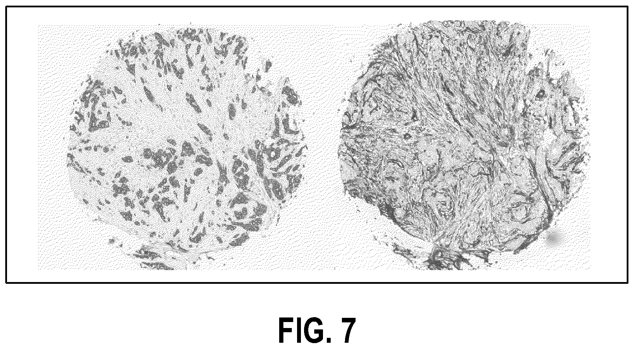

FIG. 7 depicts the results of two triplex IHC assays in which a tissue is assayed for all six markers. On the left is an image depicting the results of a first triplex assay (a first panel) performed on a breast tissue sample obtained from an individual having a breast cancer. On the first panel, S100 is visualized via a blue chromogen, AE1/3 is visualized via a red chromogen, and desmin is visualized via a green chromogen. On the right is an image depicting the results of a second triplex assay (a second panel), performed on the same tissue sample. On the second panel, SMA is visualized via a blue chromogen, CD34 is visualized via a red chromogen, and CD45 is visualized via a green chromogen. Red indicates AE1/3 cytokeratin positive core.
FIG. 8 depicts further results of two triplex IHC assays in which a tissue is assayed for all six markers. On the left is an image depicting the results of a first triplex assay (a first panel) performed on a breast tissue sample obtained from an individual having a breast cancer. On the first panel, S 100 is visualized via a blue chromogen, AE1/3 is visualized via a red chromogen, and desmin is visualized via a green chromogen. On the right is an image depicting the results of the results of a second triplex assay (a second panel), performed on the same tissue sample. On the second panel, SMA is visualized via a blue chromogen, CD34 is visualized via a red chromogen, and CD45 is visualized via a green chromogen. Red indicates AE1/3 cytokeratin positive core.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

### I. DEFINITIONS

Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

All patents and publications, including all sequences disclosed within such patents and publications, referred to herein are expressly incorporated by reference.

The headings provided herein are not limitations of the various aspects or embodiments of the invention. Accordingly, the terms defined immediately below are more fully defined by reference to the specification as a whole.

Unless defined otherwise, all technical and scientific terms used herein have the same meaning as commonly understood by one of ordinary skill in the art to which this invention belongs. The practice of the present technology will employ, unless otherwise indicated, conventional techniques of tissue culture, immunology, molecular biology, microbiology, cell biology, and recombinant DNA, which are within the skill of the art. See, *e.g*., Sambrook and Russell eds. (2001) Molecular Cloning: A Laboratory Manual (Table of Contents available at www.cshlpress.com/pdf/sample/2013/MC4/MC4FM.pdf).

All numerical designations, *e.g*., pH, temperature, time, concentration, and molecular weight, including ranges, are approximations which are varied (+) or (-) by increments of 1.0 or 0.1, as appropriate, or alternatively by a variation of +/-15%, or alternatively 10%, or alternatively 5%, or alternatively 2%. It is to be understood, although not always explicitly stated, that the reagents described herein are merely exemplary and that equivalents of such are known in the art.

It is to be inferred without explicit recitation and unless otherwise intended, that when the present technology relates to a polypeptide, protein, polynucleotide or antibody, an equivalent or a biologically equivalent of such is intended within the scope of the present technology.

A "diagnostic marker" is a specific biochemical in the body which has a particular molecular feature that makes it useful for detecting a disease, measuring the progress of disease or the effects of treatment, or for measuring a process of interest.

The term "epitope" as used herein is defined as a specific molecular structure or region on an antigen molecule that is recognized and bound by an antibody or the antigen-binding site of a T cell receptor. Epitopes can vary in size and composition, including short peptide sequences, protein domains, carbohydrate moieties, or other molecular features.An antigen can have one or more epitopes. One skilled in the art understands that generally the overall three-dimensional structure or the specific linear sequence of the molecule can be the main criterion of antigenic specificity.

A "subject" of diagnosis or treatment is a plant or animal, for example a mammal, including a human. Non-human animals subject to diagnosis or treatment include, for example, livestock and pets. The term "individual" or "patient" is used interchangeably.

As used herein, the term "tissue section" refers to a piece of tissue that has been obtained from a subject and mounted on a planar surface, *e.g.,* a microscope slide. The sample may be fixed and/or sectioned as desired. A "tumor tissue sample" or "tumor tissue biopsy sample" includes cells derived from a tumor in a subject, *e.g*., a human subject having a malignancy. Such tissue samples are sometimes referred to simply as a "biopsy".

As used herein, the term "formalin-fixed paraffin embedded (FFPE) tissue section" refers to a piece of tissue, *e.g.,* a biopsy that has been obtained from a subject, fixed in formaldehyde (*e.g.,* 3%-5% formaldehyde in phosphate buffered saline) or Bouin solution, embedded in wax, cut into thin sections, and then mounted on a planar surface, *e.g.,* a microscope slide.

"Molecule of interest," "target molecule," "target," "target antigen," "biomarker," and "analyte of interest" are interchangeably used terms, each referring to a molecule for which the presence, location and/or concentration is to be determined. Examples of molecules of interest include proteins (including peptides) and nucleic acid sequences.

The term "staining" includes binding a target (*e.g.,* an antigen) in a cellular sample with a target-specific binding agent (*e.g.*, an antibody) and then detecting the presence of the target-specific binding agent on the cells of the cellular sample using a detectable label or chromogen. The detectable label can be directly conjugated to the target-specific binding agent (*e.g.*, a primary antibody) or may be conjugated to a secondary reagent that binds specifically to an unlabeled target-specific reagent (*e.g.*, a secondary antibody). In some cases, the target-specific reagent is itself detectable, and thus no additional attached label is needed.

A "chromogen" or "chromogenic compound" and the like is a substance that can be converted into a colored compound under specific conditions, *e.g*., when acted upon by an enzyme or under specific chemical/reaction conditions. Examples of enzyme-substrate combinations include: (i) Horseradish peroxidase (HRP) with hydrogen peroxidase as a substrate, where the hydrogen peroxidase oxidizes a dye precursor; and ii) alkaline phosphatase (AP). Numerous other enzyme-substrate combinations are available to those skilled in the art. For a general review of these, see U.S. Pat. Nos. 4,275,149 and 4,318,980.

As used herein, the term "target-specific binding agent" or "binding moiety" means any agent that specifically binds to a target or analyte of interest, *e.g*., a target of interest that is present in a tissue section as described herein (*e.g.,* a polypeptide). Examples of target-specific binding agents include antibodies, receptors, and ligands, or target-binding fragments thereof, polynucleotide probes, and the like.

As used herein, the term "multiplexing" refers to using more than one label, stain, and/or chromogen for the simultaneous or sequential detection and measurement of a target in a sample, *e.g.,* a tissue section. As used herein, the term "triplexing" refers to using three labels, stains, and/or chromogens for the simultaneous or sequential detection and measurement of a target in a sample, *e.g.,* a tissue section.

As used herein, the terms "antibody" and "immunoglobulin" are used interchangeably and such terms are well understood by those in the field. Those terms refer to a protein consisting of one or more polypeptides that specifically binds an antigen. One form of antibody constitutes the basic structural unit of an antibody. This form is a tetramer and consists of two identical pairs of antibody chains, each pair having one light and one heavy chain. In each pair, the light and heavy chain variable regions are together responsible for binding to an antigen, and the constant regions are responsible for the antibody effector functions. These terms also include fragments of antibodies which retain specific binding to antigen or target, including, but not limited to, Fab, Fv, scFv, and Fd fragments, chimeric antibodies, humanized antibodies, single-chain antibodies, bi-specific hybrid antibodies, and fusion proteins comprising an antigen-binding portion of an antibody and a non-antibody protein.

As used herein, the terms "primary antibody" and "secondary antibody" refer to different antibodies, where a primary antibody is a polyclonal or monoclonal antibody from one species (rabbit, mouse, goat, donkey, etc.) that has specific binding for an antigen (*e.g.,* a biomarker) in a sample (*e.g.,* a human tissue sample) under study, and a secondary antibody is an antibody (usually polyclonal) from a different species that has specific binding for the primary antibody, e.g., in its Fc region.

The term "specific binding" refers to the ability of a binding agent to preferentially bind to a particular analyte that is present in a homogeneous mixture of different analytes. In certain aspects, a specific binding interaction will discriminate between desirable and undesirable analytes in a sample, in some aspects more than about 10 to 100-fold or more (*e.g*., more than about 1000- or 10,000-fold).

### II. Multiplex Methods for Determing a Primary Origin of a Cancer of Unknown Primary (CUP)

Disclosed are methods and systems for determining a primary origin of a cancer of unknown primary (CUP). FIG. 1 depicts the general steps involved in from initial presentation of a malignancy (50) to further classifying a malignancy (54). Referring to FIG. 1, diagnosing an unknown malignant lesion typically involves a series of steps. Initially, a patient presents with a malignancy (50) of unknown origin (also referred to as a CUP). The presentation of malignancy (50) generally occurs when a patient presents with symptoms suggestive of malignancy, such as a suspicious lump, abnormal growth, or other concerning signs. A biopsy (51) may be performed to obtain a tissue sample from the suspected lesion. This biopsy (51) can be obtained through various methods, including needle biopsy, core biopsy, or surgical excision, depending on the size and location of the lesion. The biopsy specimen then undergoes histological examination. During this process, the tissue sample may be fixed, embedded in paraffin wax, sliced into thin sections, and stained with hematoxylin and eosin (H&E) or other histological stains. The stained slides may then be examined under a microscope by a pathologist to assess the morphology of the cells and tissues. In cases of CUP, the morphology of the cells may not provide a definitive diagnosis and result in an inconclusive morphology result (51). When a morphology result is inconclusive an immunohistochemical (IHC) analysis (100) (as shown in FIG. 2 and described herein) may be carried out to provide an initial indication of the broad type of cancer. Following the IHC assay (100) in which slides are inspected (53) for staining of the target antigens, a follow up assay may be carried out to further classify the malignancy (54). Exemplary IHC assays and related methods are provided herein.

### Tissue Sample and Preparation of Tissue Section

Tissue samples and preparation of tissue sections may be carried out according to methods known in the art. For example, tissue may be obtained from a subject as a result of routine screening or from a subject that has, or is suspected of having a CUP, and may be isolated from an individual, *e.g*., from a soft tissue, tumor, or from a bodily fluid. In other aspects, the sample may be from a cell culture that is grown *in vitro* from a sample obtained from an individual. In aspects, the sample may be obtained from brain, adrenal gland, skin, lung, spleen, kidney, liver, spleen, lymph node, bone marrow, bladder stomach, small intestine, large intestine, and/or muscle. Tissue samples may be obtained using any method known in the art, for example, via tissue biopsy, scrape, lavage, and combinations thereof.

Preparation of tissue sections are known. In one aspect, the tissue section used in the disclosed methods may comprise a tissue sample that has been formalin fixed, paraffin embedded (FFPE) as described, for example, in U.S. Patent Application Publication No. 2020/0049599. In brief, a tissue sample may be contacted with a formalin fixation buffer, dehydrated, and embedded in paraffin. Sections may then be cut, for example in three-micron thick sections and placed on a glass slide for analysis using the disclosed IHC methods and systems.

### Target Antigens

Cancer diagnosis, differential diagnosis and classification are commonly based, in part, on immunohistochemistry analysis. As discussed above, in view of the large number of available biomarkers for determination of the origin of a CUP, the use of each known and available biomarker is not practical due to the limited tissue sample that is generally available. As such, the disclosed methods employ a two-panel assay, each panel employing a triplex assay in which three IHC assays are carried out per panel, allowing for the detection of six biomarkers using two tissue sections, thereby reducing the amount of tissue sample needed to perform the assay. With reference to FIG. 2 the disclosed methods provide for an Immunohistochemistry Assay Protocol (100), which comprises a first IHC assay (Layer 1 Staining Protocol (110)) for detection of a first target antigen, a second IHC assay (Layer 2 Staining Protocol (120)) for detection of a second target antigen, and a third IHC assay (Layer 3 Staining Protocol (130)) for detection of a third target antigen, as described herein. In particular, the disclosed methods provide for the detection of the following six biomarkers: smooth muscle actin, S100, CD45, CD34, desmin, and cytokeratin. Visualization of the stained tissues following completion of the IHC assays can then be used to aid in classification of the CUP.

In one aspect, the target of the first IHC Assay, the second IHC assay, or the third IHC assay is Smooth Muscle Actin. Smooth Muscle Actin is a biomarker that may be used to identify pericytes, myoepithelial cells, smooth muscle cells and myofibroblasts in normal, reactive or neoplastic tissue. In one aspect, the target of the first IHC Assay, the second IHC assay, or the third IHC assay is S100. S100 is a marker of Schwann cells and melanocytes, which may be used for evaluating nerve sheath tumors and melanoma. In one aspect, the target of the first IHC Assay, the second IHC assay, or the third IHC assay is CD45. CD45, which is also known as Leukocyte Common Antigen (LCA), is a ubiquitous cell surface marker of nucleated hematopoietic cells. In one aspect, the target of the first IHC Assay, the second IHC assay, or the third IHC assay is CD34. CD34 is a marker of vascular endothelial cells which is a useful marker of neoangiogenesis. In one aspect, the target of the first IHC Assay, the second IHC assay, or the third IHC assay is desmin. Desmin is expressed in neoplasms with myogenic differentiation (including rhabdomyosarcoma, rhabdomyoma, leiomyosarcoma, leiomyoma, smooth muscle and rhabdomyoblastic elements in other tumors). In one aspect, the target of the first IHC Assay, the second IHC assay, or the third IHC assay is cytokeratin. Cytokeratin may be detected via a mixture of two different clones of anticytokeratin (CK) monoclonal antibodies (AE1 and AE3), which functions as a broad-spectrum cytokeratin marker. Immunoreactivity is observed in epithelia and most carcinomas (i.e., tumors of epithelial origin), with cytoplasmic and membranous positivity. The aforementioned biomarkers may be detected in any order; that is, any biomarker may be detected in any phase of the IHC protocol as shown in FIG. 2, in conjunction with any of the disclosed chromogens, provided each of the three chromogens contacted with a tissues section can be visualized and distinguished from each other to allow for visualization of each of the three biomarkers in a single tissue sample.

### Immunohistochemistry Methods

The disclosed methods generally use known principles of immunohistochemistry, in which a target antigen is bound to a binding moiety, in particular an antibody. IHC assays may be used to detect biomarkers within the context of intact cells by labeling binding agents that specifically bind to the biomarker for visualization of the biomarker. By identifying the biomarker in the context of the overall tissue structure and cellular environment, spatial relationships between the biomarkers and other morphological or molecular features of the cell or tissue sample can be elucidated. Exemplary methods of immunohistochemistry are described in, for example, Magaki S, Hojat SA, Wei B, So A, Yong WH. An Introduction to the Performance of Immunohistochemistry. Methods Mol Biol. 2019;1897:289-298. doi:10.1007/978-1-4939-8935-5_25. Exemplary steps of IHC can be summarized as follows: antigen retrieval, addition of primary antibody, application of a secondary antibody that binds the primary antibody, and addition of a detection reagent to localize the primary antibody.

In brief, referring to FIG. 2, the first step in IHC is typically epitope retrieval (111), in which a tissue sample is pretreated to retrieve antigens masked by fixation and make them more accessible to antibody binding. Formalin fixation and paraffin embedding can mask antigenic sites within the tissue. Epitope retrieval methods, such as heat-induced epitope retrieval or enzymatic digestion, may be employed to unmask these sites and make the antigens accessible to antibodies for use in the disclosed IHC methods. Antigen retrieval methods may depend, in part, on the specific target antigen and antibody, but generally involve the breaking of protein cross-links caused by fixation, such as formalin, through chemical or physical means. Exemplary physical treatments include, but are not limited to, heat and ultrasound while exemplary chemical methods include, but are not limited to, enzyme digestion and denaturant treatment. One or both may be used to pretreat a tissue sample. Following epitope retrieval step (111), the tissue may be optionally blocked (not shown), for example via application with serum proteins or bovine serum albumin (BSA), to reduce nonspecific binding of a primary antibody. Following epitope retrieval (111), an optional hydrogen peroxide treatment step (112) may be carried out to block endogenous peroxidase activity and minimize nonspecific staining. Following epitope retrieval (111), and referring to the Layer 1 Staining Protocol (110) shown in FIG. 2, a primary antibody step (113) is carried out. In primary antibody step (113), a primary antibody specific to the biomarker (target) of interest is applied to the tissue section and incubated for a time and at a temperature sufficient to permit binding of the primary antibody to its target antigen in the tissue sample. For example, the primary antibody may be monoclonal or polyclonal, and may be of any species, for example mouse or rabbit. Following an incubation period, a wash step is carried out (not shown) in which excess, non-bound primary antibody is removed by washing the tissue section with a buffer solution. Removal of excess, unbound primary antibody reduces background staining.

In general, the disclosed methods employ the detection, via an IHC assay, of six biomarkers, which may be used to aid in the classification of a CUP. In one aspect, the disclosed method comprises carrying out a plurality of IHC assays, the IHC assays employing a plurality of primary antibodies, each of the plurality of antibodies being specific for the following biomarkers: smooth muscle actin, S100, CD45, CD34, desmin, and cytokeratin.

In one aspect, the method may comprise:
performing, on a first tissue section, a first IHC assay, a second IHC assay, and a third IHC assay, each of said first, second, and third IHC assay detecting a different biomarker selected from smooth muscle actin, S100, CD45, CD34, desmin, and cytokeratin;
performing, on a second tissue section, a fourth IHC assay, a fifth IHC assay, and a sixth IHC assay, each of said fourth, fifth, and sixth IHC assay detecting a different biomarker selected from smooth muscle actin, S100, CD45, CD34, desmin, and cytokeratin;
wherein each said biomarker are detected.

In one aspect, the binding moiety of the primary antibody application step may be, for example, a polyclonal antibody having having specific binding for a biomarker selected from smooth muscle actin, S100, CD45, CD34, desmin, or cytokeratin. The binding moiety of the primary antibody application step may be, for example, a monoclonal antibody having specific binding for a biomarker selected from smooth muscle actin, S100, CD45, CD34, desmin, or cytokeratin. In other aspects, the binding moiety may be, for example, a polyclonal antibody having binding specificity for a biomarker selected from smooth muscle actin, S100, CD45, CD34, desmin, or cytokeratin. The six above-described biomarkers may be detected in any arrangement and are not intended to be limited by the description or examples herein.

In one aspect, a first IHC assay (for example as shown in Layer 1 Staining Protocol (110), of FIG. 2) a first IHC assay may comprise contacting a tissue sample with a first primary antibody in a primary antibody step, the first primary antibody being specific to a biomarker selected from smooth muscle actin, S100, CD45, CD34, desmin, and cytokeratin.

In one aspect, a second IHC assay (for example as shown in Layer 2 Staining Protocol (120), of FIG. 2) a second IHC assay may comprise contacting a tissue sample with a second primary antibody in a second primary antibody step, the second primary antibody being specific to a biomarker selected from smooth muscle actin, S100, CD45, CD34, desmin, and cytokeratin.

In one aspect, a third IHC assay (for example as shown in Layer 3 Staining Protocol (130), of FIG. 2) a third IHC assay may comprise contacting a tissue sample with a third primary antibody in a primary antibody step, the third primary antibody being specific to a biomarker selected from smooth muscle actin, S100, CD45, CD34, desmin, and cytokeratin.

In one aspect, any of the first, second, or third IHC assays may further comprisean optional post-primary linker step which is carried out following application of the primary antibody. A post-primary linker may be used to bridge the primary antibody to the detection system and may be used to facilitate the binding of the secondary antibody for to the primary antibody for further amplification of the signal. Suitable post-primary linkers will be understood by one of ordinary skill in the art.

Following the primary antibody application and optional post-primary linker application, a secondary antibody may be applied to the tissue section in a secondary antibody application step. The secondary antibody is specific to and binds to the primary antibody, amplifying the signal and allowing for visualization of the target antigen. In general, the secondary antibody is targeted against the immunoglobulin of the species in which the primary antibody is produced. To visualize an antigen-antibody interaction, either the primary antibody used in primary antibody step or the secondary antibody used in secondary antibody step is labeled. In one aspect, the secondary antibody is labeled, allowing for signal amplification and use with more than one primary antibody. In one aspect, the label is an enzyme selected from horseradish peroxidase or alkaline phosphatase, which produce a colored product after incubation with a chromogenic substrate as applied in step chromogen application step. Further descriptions of exemplary IHC assays are provided herein.

### Chromogens

Chromogenic substrates are widely used for immunohistochemistry and suitable chromogens will be readily appreciated by one of ordinary skill in the art. Chromogenic substrates generally precipitate when activated by the appropriate enzyme, the enzyme converting a chromogenic substance from a soluble reagent into an insoluble, colored precipitate upon contact with the enzyme. The precipitate then deposits on the tissue at the site of antibody binding allowing for visualization of the biomarker. Chromogen intensity is generally directly proportional to the concentration of the biomarker present in the sample, providing further clinical information.

The disclosed methods may employ any chromogen known in the art sufficient to allow for distinguishing a first, second, and third chromogen in a single tissue sample. For example, in one aspect, a first IHC assay may be carried out to form a precipitate of a first color on the tissue sample, a second IHC assay may be carried out to form a precipitate of a second color on the tissue sample, and a third IHC assay be carried out to form a precipitate of a third color on the tissue sample. Exemplary chromogen colors include blue, red, green, and brown. In aspects, the use of the red and green chromogens, when used to detect each of the three target antigens as described, may create a fourth color (purple) which can further be used for diagnostic purposes.

In one aspect, three IHC assays are performed per tissue sample, *i.e.*, a triplex assay is carried out in which the three IHC assays performed on a single tissues sample employs three distinct chromogens. For example, a combination of three of the following: blue, brown, red, and green may be used for detection of the three biomarkers per tissue sample, representing three IHC assays. The three distinct chromogens used in the disclosed methods allow for visualization of the presence and/or quantification of the concentration of at least three distinct targets in a tissue sample. For example, in one aspect, the first, second, and third IHC assay as described above may employ a blue chromogen, a red chromogen, and a green chromogen, as depicted in, for example, FIGS. 8 and 9.

In one aspect, the disclosed methods may employ a blue chromogen. Blue chromogens suitable for the disclosed methods will be appreciated by one of ordinary skill in the art. For example, following the application of a primary antibody and secondary antibody coupled to an enzyme as described herein, a chromogen solution comprising a blue chromogen may be applied. In one aspect, either the first chromogen, second chromogen, or third chromogen is a chromogen producing a blue signal. In one aspect, the first IHC assay performed on a tissue section may comprise contacting the tissue section with a primary antibody (for example, a mouse or rabbit monoclonal antibody), wherein the primary antibody is specific to target molecule selected from smooth muscle actin (the antibody being referred to as anti-SMA), S100 (the antibody being referred to as anti-S100), CD45 (the antibody being referred to as anti-CD45), CD34 (the antibody being referred to as anti-CD34), desmin (the antibody being referred to as anti-desmin), and cytokeratin (the antibody being referred to as anti-cytokeratin). Following a wash step, a secondary antibody specific for the primary antibody is then applied to the tissue section for a second incubation period. In one aspect, the secondary antibody is an anti-species antibody such as anti-mouse or anti-rabbit. In one aspect, the secondary antibody may be conjugated to an enzyme that is capable of precipitating a first chromogen. In this aspect, the first chromogen is a blue chromogen, which is applied to the tissue section. In the presence of the target molecule, the blue chromogen is precipitated as a result of contact with the enzyme conjugated with the secondary antibody. In further aspects, an α-mouse IgG linker (for example may be contacted with the tissue prior to the secondary antibody for further amplification of the signal produced by the chromogen, as will be readily understood by one of ordinary skill in the art. An exemplary blue chromogen system employs a blue salt and a naphthol phosphate. For example, the blue salt may comprise a diazonium salt of Fast Blue RR such as 4-Benzoylamino-2,5-dimethoxybenzenediazonium chloride hemi(zinc chloride) salt (CAS: 14726-29-5) also referred to as RR Salt and Azoic Diazo No. 24, available from Sigma-Adrich^{®}. The naphthol phosphate may be one selected from naphthol AS phosphate, naphthol AS-OL phosphate, naphthol AS-E phosphate, naphthol AS-MX phosphate, naphthol AS-TR phosphate, naphthol AS-BI phosphate, naphthol AS-BS phosphate and naphthol AS-GR phosphate, the salts and hydrates thereof and mixtures thereof. In one aspect, the naphthol phosphate is N-(2,4,-dimethylphenyl)-3-phosphonooxy)-2-naphthalenecarboxamide, or (Naphthol AS-MX), CAS:1596-56-1. Each of the two compositions may be supplied individually, and may be supplied in a buffer composition. The compositions may further comprise one or more of sodium chloride, magnesium chloride, a buffer, a preservative, and antimicrobial, and combinations thereof. In one aspect, a blue chromogen composition is disclosed, the composition comprising RR Salt and Naphthol AS-MX. Applying the IHC methods described herein, the blue precipitate deposits on the treated sample, allowing for visualization of the target, as identified by a blue color on the sample.

In one aspect, the disclosed methods may employ a brown chromogen. Brown chromogens suitable for the disclosed methods will be appreciated by one of ordinary skill in the art. For example, following the application of a primary antibody and secondary antibody coupled to an enzyme as described herein, a chromogen solution comprising a brown chromogen may be applied. In one aspect, either the first chromogen, second chromogen, or third chromogen is a chromogen producing a brown signal. In one aspect, an IHC assay performed on a tissue section may comprise contacting the tissue section with a primary antibody (for example, a mouse or rabbit monoclonal antibody), wherein the primary antibody is anti-SMA, anti-S100, anti-CD45, anti-CD34, anti-desmin, or anti-cytokeratin. Following a wash step, a secondary antibody specific for the primary antibody is then applied to the tissue section for a second incubation period. In one aspect, the secondary antibody is an anti-species antibody such as anti-mouse or anti-rabbit. In one aspect, the secondary antibody may be conjugated to an enzyme that is capable of precipitating a chromogen. In this aspect, the chromogen is a brown chromogen, which is applied to the tissue section. In the presence of the target molecule, the brown chromogen is precipitated as a result of contact with the enzyme conjugated with the secondary antibody. In further aspects, an α-mouse IgG linker (for example may be contacted with the tissue prior to the secondary antibody for further amplification of the signal produced by the chromogen, as will be readily understood by one of ordinary skill in the art. An exemplary brown chromogen is DAB, or 3,3'-diaminobenzidine. In this aspect, the enzyme used is HRP, which catalyzes the oxidation of DAB in the presence of hydrogen peroxide. This reaction results in the formation of a brown-colored insoluble precipitate at the site of enzyme activity. Upon visualization, the presence of the antigen (smooth muscle actin, S100, CD45, CD34, desmin, or cytokeratin) is indicated by brown staining at the location of the target protein.

The disclosed methods may employ a red chromogen. Red chromogens suitable for the disclosed methods will be appreciated by one of ordinary skill in the art. For example, following the application of a primary antibody and secondary antibody coupled to an enzyme as described herein, a chromogen solution comprising a red chromogen may be applied. In one aspect, either the first chromogen, second chromogen, or third chromogen is a chromogen producing a red signal. The IHC assay may comprise contacting the tissue section with a primary antibody (for example, a mouse or rabbit monoclonal antibody) wherein the primary antibody is anti-SMA, anti-S100, anti-CD45, anti-CD34, anti-desmin, or anti-cytokeratin, and the primary antibody is incubated with the tissue section for a first incubation period. Following a wash step, a secondary antibody is then applied to the tissue section for a second incubation period. In one aspect, the secondary antibody is an anti-species antibody such as anti-mouse or anti-rabbit. In one aspect, the secondary antibody may be conjugated to an enzyme that is capable of precipitating a second chromogen. In this aspect, the second chromogen is a red chromogen, which is applied to the tissue section. In the presence of the target molecule, the red chromogen is precipitated as a result of contact with the enzyme conjugated with the secondary antibody. In further aspects, an α-mouse IgG linker may be contacted with the tissue prior to the secondary antibody for further amplification of the signal produced by the chromogen. An exemplary red chromogen may be Permanent Red (also known as Fast Red), Nathol AS-MX Phosphate, Bromo-Chloro-Indolyl Phosphate (BCIP) with Nitroblue Tetrazolium (NBT), New Fuchin, Vector Red, or combinations thereof. In this aspect, the enzyme is alkaline phosphatase (AP), which causes the formation of a red-colored insoluble precipitate at the site of enzyme activity. Upon visualization, the presence of the antigen (smooth muscle actin, S100, CD45, CD34, desmin, or cytokeratin) is indicated by red staining at the location of the target protein.

The disclosed methods may employ a green chromogen. Green chromogens suitable for the disclosed methods will be appreciated by one of ordinary skill in the art. For example, following the application of a primary antibody and secondary antibody coupled to an enzyme as described herein, a chromogen solution comprising a green chromogen may be applied. In one aspect, either the first chromogen, second chromogen, or third chromogen is a chromogen producing a green signal. In one aspect, the IHC assay performed on a tissue section may comprise contacting the tissue section with a primary antibody (for example, a mouse or rabbit monoclonal antibody), wherein the primary antibody is anti-SMA, anti-S100, anti-CD45, anti-CD34, anti-desmin, or anti-cytokeratin. Following a wash step, a secondary antibody is then applied to the tissue section for a second incubation period. In one aspect, the secondary antibody is an anti-species antibody such as anti-mouse or anti-rabbit. In one aspect, the secondary antibody may be conjugated to an enzyme that is capable of precipitating a second chromogen. In this aspect, the chromogen is a green chromogen, which is applied to the tissue section. In the presence of the target molecule, the green chromogen is precipitated as a result of contact with the enzyme conjugated with the secondary antibody. An exemplary green chromogen may be 4-Chloro-1-Naphthol (4-CN), Leucocrystal Violet, Fast Green FCF, Malachite Green, Rhodamine Green carboxylic acid succinimidyl ester (DY-505),], or combinations thereof. In this aspect, the enzyme may be HRP, which causes the formation of a green-colored insoluble precipitate at the site of enzyme activity. Upon visualization, the presence of the antigen (smooth muscle actin, S100, CD45, CD34, desmin, or cytokeratin) is indicated by green staining at the location of the target protein.

### Counterstain

The disclosed methods may further comprise a cytochemical staining procedure for further visualization of cells or cell compartments such that their structures can be more readily visualized under a microscope, such staining procedure being carried out in Post-Processing step (140). For example, a counterstain may be used prior to cover-slipping to render the immunohistochemical stain more distinct. Such staining procedures are known to those of skill in the art and may comprise, for example, staining for acidophilic or basophilic structures, of subcellular regions (*e.g*. the nucleus, the mitochondria, the golgi, the cytoplasm etc.), of specific molecules (of chromosomes, of lipids, of glycoproteins, of polysaccharids etc.) in the cytological specimens. Fluorescence dyes such as DAPI, Quinacrin, Chromomycin, etc. may be employed. Furthermore, chromogenic dyes such as Azan, Acridin-orange, Hematoxylin, Eosin, Sudan-red, Thiazin-stains (Toluidin-blue, Thionin) may be applied. In other aspects, staining procedures such as Pap-staining, Giemsa-staining, Hematoxylin-Eosin staining, van-Gieson staining, Schiff-staining (using Schiff reagent), staining procedures employing precipitation of metals (such as *e.g.* of silver in staining procedures employing Silver Nitrate) or insoluble stains such as *e.g.* of Turnbulls-blue (or other insoluble metal cyanides), etc. may be used.

### Use of the Disclosed Systems and Methods for Diagnosis and Treatment

In one aspect, the disclosed methods may be used to inform a treating physician, based on the detection of markers in a tissue sample obtained from an individual, of the origin of cancer in an individual identified as having a CUP, and/or for development of a personalized dosage or treatment regimen for the individual. The disclosed methods may be used in conjunction with other patient-derived data, which may include, for example, additional biomarker tests, such as those described in U.S. Patents 9,234,892, 10,041,949 and 10,370,698, patient symptoms, patient history, assessment of cellular structure, and the like.

In one aspect, the disclosed methods may be used in the process of diagnosis, stage determination, treatment identification, or a subset determination of a cancer origin in and individual having a CUP. The disclosed methods may be used to determine that an origin of cancer is, for example, one or more of adenocarcinoma, squamous cell carcinoma, undifferentiated or poorly differentiated carcinoma, neuroendocrine tumors, melanoma, lymphoma, sarcoma, germ cell tumors, thyroid cancer, carcinoid tumors, renal cell carcinoma, hepatocellular carcinoma, bladder cancer, breast cancer, ovarian cancer, testicular cancer, and/or pancreatic cancer.

In one aspect, the disclosed methods may be used to obtain biomarker data from a tissue section comprising a tissue sample obtained from an individual, which may be used in the process of selecting one or more drugs and/or dosage (amount, length of administration, etc.) for treatment of a cancer in the individual. In other aspects, the methods may be used to modify or personalize a treatment plan based upon the patient's individual biomarker profile, based on a determination of the presence, absence, or level of one or more biomarkers as disclosed herein, which is further used to determine the origin of cancer.

In one aspect, the disclosed methods may be used, alone or in combination with other patient-derived data, to determine an appropriate treatment plan for an individual, wherein the disclosed methods are used to determine the origin of a cancer, and, based on the determination of the cancer origin, a therapy is selected from chemotherapy, immunotherapy, a targeted therapy directed to a dysregulated signaling pathway, radiation therapy, stem cell transplant, or combinations thereof.

For example, in one aspect the disclosed methods may be used to determine the broad tumor type of a CUP. In one aspect, following detection of the six biomarkers disclosed herein on a tissue sample, a broad tumor type is determined. The broad tumor type may be one or more selected from carcinoma, melanoma, lymphoma and/or sarcoma. The two triplex assays as disclosed herein may be used as a first line indication to rule out broader cancer types such as carcinoma, melanoma, lymphoma and/or sarcoma. For example, in one aspect, the first triplex assay comprises detection of S100 using a blue chromogen, cytokeratin using a red chromogen, and desmin using a green chromogen. n this aspect, blue staining indiciating S100 positive tissue is likely to be melanoma in origin. Red staining indicating cytokeratin positive cells indicates that the primary cancer islikely to be carcinoma in origin. Green staining indicating desmin positive tissue,indicates a sarcoma subtype origin. In one aspect, the second triplex panel is carried out, comprising detection of Smooth Muscle Actin using a blue chromogen, CD34 using a red chromogen, and CD45 using a green chromogen. In this aspect, blue staining indicates Smooth Muscle Actin positive tissue and indicates that the cancer is likely of sarcoma subtype origin. Red staining indicates CD34 positive tissue and indicates cancer of a sarcoma subtype origin. Green staining indicates CD45 positive tissue and indicates that the cancer is likely a lymphoma in origin.

### III. Example Slide-Staining Instrument

In one aspect, disclosed are methods for the preparation of samples for IHC detection methods which may employ, in whole or in part, the slide-staining instrument (10) described herein. It should be understood that other instruments may be used to carry out the disclosed methods, for example that described in U.S. Patent 11493411, U.S. Patent Publication No. 2021/0293670, and U.S. Patent Publication No. 2023/0022299. FIG. 3 shows an example of a slide-staining instrument (10) that is generally configured to automatically stain tissue mounted on microscope slides. Slide-staining instrument (10) includes a group fluid dispenser (12) (also referred to as an aspirating probe) mounted to a robotic arm (14) and a plurality of slide staining assemblies (40) disposed beneath robotic arm (14). As will be described in greater detail below, robotic arm (14) and slide staining assemblies (40) are generally configured to operate cooperatively to automatically dispense reagents, for example, IHC assay reagents, stain, probes, enzymes, and the like, onto one or more microscope slides disposed within each slide staining assembly (40).

To supply reagents, slide-staining instrument (10) includes a bulk container rack (20) and a reagent platform (30). Bulk container rack (20) is configured to hold a plurality of bulk containers (22). Bulk containers (22) may be configured to contain a relatively large quantity of reagent that may be communicated to group fluid dispenser (12) and/or each slide staining assembly (40). Examples of reagents that may be stored in bulk containers (22) may include, for example, formalin solutions, wash solutions, deionized water, buffered solutions such as phosphate buffered saline (PBS), and/or alcohol, and additional reagents useful to carrying out the disclosed methods. Additionally, one or more of bulk containers (22) may be configured to contain waste fluids communicated from group fluid dispenser (12) and/or each slide staining assembly (40). In some examples, such waste fluids may be separated by slide-staining instrument (10) into bulk waste and hazardous waste. Thus, separate bulk containers (22) may be included for bulk waste and hazardous waste. A plurality of syringe pumps (24) may be included proximate bulk containers (22) to communicate fluid to or from bulk containers (22) relative to other portions of slide-staining instrument (10).

Reagent platform (30) is generally configured to receive one or more reagent trays (32). Each reagent tray (32) is configured to hold a plurality of reagent containers (34). Reagent containers (34) may be configured to contain a relatively small quantity of reagent for communication to group fluid dispenser (12) and/or slide staining assembly (40). Each reagent tray (32) may include a particular predetermined combination of reagent containers (34) to form a predefined reagent system. Such predefined reagent systems may be ready-to-use and are discarded upon exhaustion during use with a particular protocol. Each reagent tray (32) is registered upon insertion into reagent platform (30). Although not shown, it should be understood that group fluid dispenser (12) may access reagent containers (34) directly. Also, one or more syringe pumps similar to syringe pumps (24) described above may be included to communicate reagents from reagent containers (34) to group fluid dispenser (12), slide staining assembly (40), and/or other portions of slide-staining instrument (10).

In use, robotic arm (14) is used to position group fluid dispenser (12) relative to slide staining assembly (40) and reagent platform (30). Robotic arm (14) may then be moved to reagent platform (30) to gather one or more reagents with group fluid dispenser (12). Optionally, one or more reagents may be mixed or otherwise manipulated in a separate area of slide-staining instrument (10). Robotic arm (14) may then move to a selected slide staining assembly (40) to dispense gathered reagents onto one or more slides disposed within the selected slide staining assembly (40).

Simultaneously with use of robotic arm (14) to dispense reagents, or separately therefrom, each slide staining assembly (40) may dispense reagents onto one or more slides disposed within a respective slide staining assembly (40). Specifically, batch fluid dispenser (46) may move along guide rail (44) to communicate one or more reagents from bulk containers (22) onto slides via syringe pumps (24) and other components associated with slide-staining instrument (10). Thus, robotic arm (14) and slide staining assemblies (40) are configured to operate cooperatively to efficiently complete staining protocols with one or more steps of a given staining protocol being performed simultaneously with one or more other steps. During the performance of such staining protocols, heaters included within each slide staining assembly (40) are used to maintain the temperature of individual slides at a selected temperature in accordance with such staining protocols.

### IV. Immunohistochemistry Assay

As described above, slide-staining instrument (10) may be configured to perform a variety of protocols where robotic arm (14) and/or slide staining assemblies (40) may automatically apply a variety of reagents to slides under various predetermined parameters. Such slide staining protocols may include a series of steps where various reagents may be applied to individual slides in a predetermined order, in a predetermined volume at predetermined temperatures for predetermined periods of time.

FIG. 2 depicts an exemplary Immunohistochemistry Assay Protocol (100). For example, a tissue section is prepared from a tissue sample obtained from an individual as described above. In one aspect, the tissue sample is a FFPE tissue section which is placed on a slide and deparaffinized using the BOND Dewax protocol, which removes paraffin wax from the tissue section before rehydration and staining. In the first phase of the Immunohistochemistry Assay Protocol (100), Layer 1 Staining Protocol (110), which comprises a first IHC assay, is carried out for detection of a first target antigen. Immunohistochemistry Assay Protocol (100) begins with incubation of the tissue section with an epitope retrieval solution, which assists in exposing the desired antigen. Exemplary epitope retrieval solutions are known in the art. An exemplary epitope retrieval solution comprises EDTA, Tris Base, Sodium Citrate, Proteinase K, Trypsin, and Pepsin. In aspects, the epitope retrieval solution is BOND Epitope Retrieval Solution 1, a ready to use, citrate-based pH 6 epitope retrieval solution for the heat-induced epitope retrieval (HIER) of formalin-fixed, paraffin-embedded tissue on the BOND automated system, which restores epitopes that have been modified by formalin fixation, allowing accessibility of the primary antibody to the epitope. The epitope retrieval solution incubation step (111) may be carried out at a temperature sufficient to expose the antigens of interest and may be carried out for a period of time ranging from about 30 seconds to about 5 minutes, or from about 1 minute to about 10 minutes, or about 2 minutes. The duration and temperature used for incubation of the reagents during the epitope retrieval solution incubation step (111) may be predetermined and programmed into the slide-staining instrument (10) and may depend on the specific epitope retrieval solution used. Following epitope retrieval solution incubation step (111), the tissue section is subjected to a hydrogen peroxide treatment (112) to quench endogenous peroxidase activity. The hydrogen peroxide treatment step (112) may employ, for example, a solution of about 3% to 4% hydrogen peroxide solution applied to the tissue section for about 30 seconds to about 5 minutes, or from about 1 minute to about 10 minutes, or about 2 minutes. First primary antibody (113) is then applied to the tissue section and incubated for a time and temperature sufficient to allow specific binding to the target. For example, the primary antibody may be incubated with the tissue section for an incubation period of about 1 to about 30 minutes or about 5 to about 25 minutes, or about 10 to about 20 minutes, or about 15 minutes. The tissue section is then subjected to post-primary linker reagent (114), for example α-mouse IgG linker localizing mouse primary antibody, for about 5 minutes to about 30 minutes, or about 10 minutes to about 20 minutes, or about 15 minutes followed by contact with a secondary antibody conjugated to an enzyme (115). In one aspect, the enzyme used in the Layer 1 Staining Protocol (110) is horseradish peroxidase. In one aspect, the enzyme used in the Layer 1 Staining Protocol (110) is alkaline phosphatase. In Layer 1 Staining Protocol (110), a first chromogen application step (116) is carried out. For example, as shown in Example 1, the chromogen of the Layer 1 staining protocol may be, for example, Fast Blue RR chromogen. First chromogen application step (116) utilizes a chromogen that is different in color from second chromogen step (124) and third chromogen step (134), and is carried out for a time and temperature sufficient to allow precipitation of the chromogen, for example, for about 5 minutes to about 20 minutes, or about 7 minutes to about 15 minutes, or about 10 minutes. Elution (117) is then carried out, in which a neutralization wash (*e.g*. EDTA based pH 9 solution) is applied to bring the tissue to neutral pH. In one aspect, elution (180) may be carried out at a temperature of from about 50°C and for a period of about 5 minutes to about 20 minutes, or about 7 minutes to about 15 minutes, or about 10 minutes. In Layer 1 Staining Protocol (110), the target of the first primary antibody may be selected from a biomarker as described herein.

In the second phase of the Immunohistochemistry Assay Protocol (100), Layer 2 Staining Protocol (120), which comprises a second IHC assay, is carried out for detection of a second target antigen. Following elution (117), Layer 2 Staining Protocol (120) is carried out on the tissue section Layer 2 Staining Protocol (120) is also shown in FIG. 2. The tissue section of Layer 1 Staining Protocol (110) described above is incubated with second primary antibody (121) for a time and temperature sufficient to allow specific binding to the target. In Layer 2 Staining Protocol (120), the target of the second primary antibody is selected from a biomarker as described herein. The tissue section is then subjected to post-primary linker reagent (122), followed by contact with a secondary antibody (123) conjugated to an enzyme. In one aspect, the enzyme used in the Layer 1 Staining Protocol (110) is horseradish peroxidase. In one aspect, the enzyme used in the Layer 1 Staining Protocol (110) is alkaline phosphatase (AP). In one aspect, as shown in Example 1, the chromogen used in the Layer 2 Staining Protocol (120) is Refine Red chromogen chromogen (for example that used in BOND Polymer Refine Red Detection, an IVD labeled red detection system, available from Leica Biosystems, Product Code DS9390). Second chromogen application step (124) utilizes a chromogen that is different in color from first chromogen step (116) and third chromogen step (134), and is carried out for a time and temperature sufficient to allow precipitation of the second chromogen, for example, for about 5 minutes to about 20 minutes, or about 7 minutes to about 15 minutes, or about 10 minutes. Elution (125) is carried out as described above.

In the third phase of the Immunohistochemistry Assay Protocol (100), Layer 3 Staining Protocol (130), which comprises a third IHC assay, is carried out for detection of a third target antigen. Following elution (125), Layer 3 Staining Protocol (130) is carried out on the tissue section. Layer 3 Staining Protocol (130) is also shown in FIG. 2. The tissue section of Layer 1 Staining Protocol (110) and Layer 2 Staining Protocol Staining Protocol (120) above is incubated with third primary antibody (131) for a time and temperature sufficient to allow specific binding to the target, as described above. In Layer 3 Staining Protocol (130), the target of the third primary antibody is selected from a biomarker as described herein. The tissue section is then subjected to post-primary linker reagent (132), followed by contact with a secondary antibody conjugated to an enzyme (133). In one aspect, the enzyme used in the Layer 1 Staining Protocol (110) is horseradish peroxidase. In one aspect, the enzyme used in the Layer 1 Staining Protocol (110) is alkaline phosphatase (AP). In Layer 3 Staining Protocol (130), a third chromogen is applied. Third chromogen application step (134) utilizes a chromogen that is different in color from first chromogen step (116) and second chromogen step (124), and is carried out for a time and temperature sufficient to allow precipitation of the third chromogen, for example, for about 5 minutes to about 20 minutes, or about 7 minutes to about 15 minutes, or about 10 minutes. In one aspect, as shown in Example 1, the chromogen used in the Layer 3 Staining Protocol (130) is a green chromogen.

The tissue section is then counterstained with a suitable counterstain in post-processing step (140), for example, hematoxylin solution, and washed to remove excess reagent. The resulting tissue section may then be visualized for the presence of the three different chromogens.

It should be understood that the above protocols may include one or more washing steps between any one or more of the steps or protocols described above prior to the dehydration step with increased concentration of alcohol ranging from 70% to 100%. Such wash steps may be performed with a variety of solutions such as deionized water, phosphate buffered saline, or other buffered solutions, or various combinations thereof.

Incubation times of any of the described steps may vary. For example, any dispense cycle in which a reagent is applied to the sample may include an incubation. Incubations are generally performed with the given slide being heated to a predetermined temperature. The incubation time may be varied depending on the desired outcome and the particular reagents (for example, the particular probe) that is used. For example, the incubation time may be about 20 minutes for a dispense cycle, or about 30 minutes for a dispense cycle, or about 60 minutes for a dispense cycle. Although particular incubation times are described, it should be understood that in other examples, either a different minimum incubation time or a different maximum incubation time may be used.

### V. Examples

The following non-limiting examples are provided to further illustrate embodiments of the invention disclosed herein. It should be appreciated by those of skill in the art that the techniques disclosed in the examples that follow represent approaches that have been found to function well in the practice of the invention, and thus may be considered to constitute examples of modes for its practice. However, those of skill in the art should, in light of the present disclosure, appreciate that many changes may be made in the specific embodiments that are disclosed and still obtain a like or similar result without departing from the spirit and scope of the invention.

### Example I.

Formalin-Fixed Paraffin-Embedded (FFPE) tissue sections of 3µm thickness were mounted onto charged slides. Slides were baked at 60°C for 60 minutes. All steps there on forth were completed on the BOND-Rx automated staining instrument (software version 7) utilizing the sequential staining method. All steps unless otherwise stated are completed at ambient temperature.

### IH Protocol, Layer 1

For panel 1 of the IHC protocol, slides are deparaffinized using the BOND Dewax protocol and heat induced epitope retrieved using BOND Epitope Retrieval Solution 1 for 20 mins at 100°C for triplex panel 1 (S100, Cytokeratin AE1/3, Desmin). Triplex panel 2 (SMA, CD34, CD45) does not require heat induced epitope retrieval within layer 1. The specimen is incubated with hydrogen peroxide for 5 minutes to quench endogenous peroxidase activity and a primary antibody (BOND Ready-to-Use reagent) of monoclonal mouse or rabbit origin is applied following an incubation of 15 minutes. A secondary reagent, α-mouse IgG linker localizing mouse primary antibodies is applied to the slide for 20 minutes and subsequently detected using a α-rabbit polymer reagent conjugated to alkaline phosphatase for 10 minutes. The Fast Blue RR chromogen is applied to the tissue for (10+10 minutes), and produces a blue precipitate, localized to the cellular compartment of the epitope in detection.

An antibody elution step is applied to the tissue before moving onto the next sequential staining round. This elution method includes a heated temperature of 50°C for 10 minutes, followed by a neutralization wash to ensure the tissue is brought back to a neutral pH.

### IHC Protocol, Layer 2

For panel 2 of the IHC protocol, a heat induced epitope retrieval step using BOND Epitope Retrieval Solution 2 for 20 mins at 100°C is used at the start of layer 2. The specimen is incubated with a primary antibody (BOND Ready-to-Use reagent) of monoclonal mouse or rabbit origin is applied following an incubation of 15 minutes. A secondary reagent α-mouse IgG linker localizing mouse primary antibodies is applied to the slide for 20 minutes and subsequently detected using a α-rabbit polymer reagent conjugated to alkaline phosphatase for 30 minutes. The Refine Red chromogen is applied to the tissue (10+5 minutes) and produces a red precipitate localized to the cellular compartment of the epitope in detection.

An antibody elution step is applied to the tissue before moving onto the next sequential staining round. This elution method includes a heated temperature of 50°C for 10 minutes, followed by a neutralization wash to ensure the tissue is brought back to a neutral pH.

### IHC Protocol, Layer 3

For panel 3 of the IHC protocol, the specimen is incubated with a primary antibody (BOND Ready-to-Use reagent) of monoclonal mouse or rabbit origin, applied for an incubation of fifteen minutes. A secondary reagent α-mouse IgG linker localizing mouse primary antibodies is applied to the slide for fifteen minutes and subsequently detected using a α-rabbit polymer reagent conjugated to horseradish peroxidase for 10 minutes. The green chromogen is applied to the tissue (10 minute incubation) and produces a green precipitate localized to the cellular compartment of the detected epitope.

Final protocol steps include a counterstain dispense of Hematoxylin solution for 1 minute at ambient temperature, followed by the application of BOND Wash Solution to allow blueing of the reagent to take place.

| **LBS Product Code** | **Antibody/Clone** | **Multiplex Panel** | **Detection Layer** | **Chromogenic Detection** |
|---|---|---|---|---|
| PA0031 | S-100/EP32 | Antibody Panel 1 | 1 | Blue |
| PA0909 | Cytokeratin/ AE1/AE3 | | 2 | Refine Red |
| PA0032 | Desmin/ DE-R-11 | | 3 | Green |
| PA0943 | Smooth Muscle Actin/ ASM-1 | Antibody Panel 2 | 1 | Blue |
| PA0212 | CD34/ QBEND/10 | | 2 | Refine Red |
| PA0042 | CD45/ X16/99 | | 3 | Green |

### Exemplary results of the assays described above are shown in FIGS. 4-8.

### V. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Exemplary combinations

### Example 1

A tissue staining method for characterization of a cancer of unknown primary (CUP) comprising: performing, on a first tissue section, a first plurality of immunohistochemistry (IHC) assays comprising a first IHC assay, said first IHC assay comprising contacting said first tissue section with a first primary antibody; a second IHC assay, said second IHC assay comprising contacting said first tissue section with a second primary antibody; and a third IHC assay, said third IHC assay comprising contacting said first tissue section with a third primary antibody; performing, on a second tissue section, a second plurality of immunohistochemistry assays comprising a fourth IHC assay, said fourth IHC assay comprising contacting said second tissue section with a fourth primary antibody; a fifth IHC assay, said fifth IHC assay comprising contacting said second tissue section with a fifth primary antibody; and a sixth IHC assay, said sixth IHC assay comprising contacting said second tissue section with a sixth primary antibody, each said IHC assay detecting a different biomarker selected from smooth muscle actin, S100, CD45, CD34, desmin, and cytokeratin.

### Example 2

The method of example 1, said primary antibody being a monoclonal antibody.

### Example 3

The method of any of examples 1 or 2, said primary antibody being a mouse or rabbit monoclonal antibody.

### Example 4

The method of any preceding example, at least one said IHC assay comprising an anti-species linker.

### Example 5

The method of example 4, said anti-species linker being an antibody specific for a primary antibody.

### Example 6

The method of any preceding example, each said IHC assay comprising a secondary antibody.

### Example 7

The method of example 6, each said secondary antibody being an anti-species monoclonal antibody.

### Example 8

The method of example 6 or 7, each said secondary antibody being an anti-species monoclonal antibody specific to said primary antibody.

### Example 9

The method of any of examples 6-8, at least one said secondary antibody being an anti-species monoclonal antibody specific to said anti-species linker.

### Example 10

The method of any of examples 7-9, said anti-species monoclonal antibody comprising an enzyme for precipitating a chromogen.

### Example 11

The method of example 10, said enzyme for precipitating a chromogen being selected from alkaline phosphatase (AP) and/or horseradish peroxidase (HRP).

### Example 12

The method of any preceding example, each of said first plurality of IHC assays comprising chromogens of a different color.

### Example 13

The method of any preceding example, each said second plurality of IHC assays comprising a chromogen of a different color.

### Example 14

The method of any preceding example, said first and/or said second plurality of IHC assays comprising at least one blue chromogen.

### Example 15

The method of any preceding example, said first and/or said second plurality of IHC assays comprising at least one brown chromogen.

### Example 16

The method of any preceding example, said first plurality and/or said second plurality of IHC assays comprising at least one red chromogen.

### Example 17

The method of any preceding example, said first plurality and/or said second plurality of IHC assays comprising at least one green chromogen.

### Example 18

The method of example 1 wherein each said IHC assay comprises contacting said tissue sample with a secondary antibody conjugated to an enzyme configured to catalyze a chromogen, said secondary antibody being specific to said primary antibody; and detecting said chromogen.

### Example 19

The method of any preceding example, each said tissue section comprising tissue obtained from an individual having, or suspected of having, a CUP.

### Example 20

The method of any preceding example, each said tissue section comprising tissue obtained from the same individual.

### Example 21

The method of any preceding example, each said tissue section comprising tissue from the same biopsy sample.

### Example 22

The method of any preceding example, each said tissue section comprising tissue from different biopsy sample.

### Example 23

The method of any preceding example, said first tissue section and said second tissue section being the same.

### Example 24

The method of any preceding example, said first tissue section and said second tissue section being different.

### Example 25

The method of any preceding example, further comprising staining said first and/or second tissue section with hematoxylin.

### Example 26

The method of any preceding example, further comprising imaging said first and/or said second tissue section.

### Example 27

The method of any preceding example, further comprising detecting a first chromogen, a second chromogen, and a third chromogen.

### Example 28

The method of any preceding example, wherein said first chromogen, said second chromogen, and said third chromogen are simultaneously detected.

### Example 29

A method for an automated multiplex IHC assay of one or more tissue samples disposed on a slide, the method comprising dispensing one or more reagents to the one or more tissue samples using one or more automated fluid dispensers in a series of staining steps associated with an IHC protocol, said reagents comprising a first reagent comprising a primary antibody specific to at least one biomarker selected from smooth muscle actin, S100, CD45, CD34, desmin, and cytokeratin; a second reagent comprising a secondary antibody specific for said first reagent; and at least one chromogen.

### Example 30

The method of example 29, further comprising dispensing one or more reagents to the one or more tissue sections using one or more automated fluid dispensers in a series of sample preparation steps associated with a sample preparation protocol.

### Example 31

The method of example 29, the primary antibody comprising a mouse monoclonal antibody.

### Example 32

The method of any of examples 29-31, the primary antibody comprising a rabbit monoclonal antibody.

### Example 33

The method of example 29-32, the secondary antibody comprising an anti-species antibody.

### Example 34

The method of any of examples 29-33, the secondary antibody comprising an anti-mouse and/or anti-rabbit antibody.

### Example 35

The method of any preceding example, wherein said method is run on an automated slide staining apparatus.

### Example 36

A kit comprising a plurality of primary antibodies, said plurality of primary antibodies comprising a primary antibody specific to smooth muscle actin, a primary antibody specific to S100, a primary antibody specific to CD45, a primary antibody specific to CD34, a primary antibody specific to desmin, and a primary antibody specific to cytokeratin.

### Example 37

The kit of example 36, further comprising a plurality of secondary antibodies, said secondary antibodies being conjugated to an enzyme selected from AP and HRP and specific to said plurality of antibodies.

### Example 38

The kit of example 37, said plurality of secondary antibodies being anti-species secondary antibodies.

### Example 39

The kit of any of examples 36-38, further comprising a plurality of anti-species linker antibodies specific to said plurality of primary antibodies.

### Example 40

The kit of any of examples 36-39, further comprising a plurality of chromogens, said plurality of chromogen comprising a brown chromogen, a red chromogen, and a green chromogen.

### Example 41

The kit of any of examples 36-40, further comprising a buffered solution.

### Example 42

Use of the kit of any of examples 36-41 in immunohistochemistry, in partiucle in a tissue staining method for characterization of a cancer of unknown primary (CUP) of any of examples 1-28.

### Conclusion

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following examples and is understood not to be limited to the details of structure and operation shown and described in the specification and drawings.

## Claims

1. A tissue staining method for characterization of a cancer of unknown primary (CUP) comprising:
(a) performing, on a first tissue section, a first plurality of immunohistochemistry (IHC) assays comprising
(i) a first IHC assay, the first IHC assay comprising contacting the first tissue section with a first primary antibody;
(ii) a second IHC assay, the second IHC assay comprising contacting the first tissue section with a second primary antibody; and
(iii) a third IHC assay, the third IHC assay comprising contacting the first tissue section with a third primary antibody;
(b) performing, on a second tissue section, a second plurality of immunohistochemistry assays comprising
(i) a fourth IHC assay, the fourth IHC assay comprising contacting the second tissue section with a fourth primary antibody;
(ii) a fifth IHC assay, the fifth IHC assay comprising contacting the second tissue section with a fifth primary antibody; and
(iii) a sixth IHC assay, the sixth IHC assay comprising contacting the second tissue section with a sixth primary antibody,
each the IHC assay detecting a different biomarker selected from smooth muscle actin, S100, CD45, CD34, desmin, and cytokeratin.

2. The method of claim 1, the primary antibody being a monoclonal antibody, in particular wherein the primary antibody is a mouse or rabbit monoclonal antibody.

3. The method of claim 1 or 2, at least one IHC assay comprising an anti-species linker, in particular wherein the anti-species linker being an antibody specific for a primary antibody.

4. The method of any one of the preceding claims, wherein each IHC assay comprises a secondary antibody, particularly wherein each the secondary antibody is an anti-species monoclonal antibody, more particularly wherein each the secondary antibody is an anti-species monoclonal antibody specific to the primary antibody, even more particularly wherein each the secondary antibody being an anti-species monoclonal antibody specific to the primary antibody and/or an anti-species monoclonal antibody specific to the anti-species linker.

5. The method of claim 4, the secondary antibody comprising an enzyme for precipitating a chromogen, in particular wherein the enzyme for precipitating a chromogen is selected from alkaline phosphatase (AP) and/or horseradish peroxidase (HRP).

6. The method of any one of the preceding claims, each of the first plurality of IHC assays comprising chromogens of a different color and/or each of the second plurality of IHC assays comprising a chromogen of a different color.

7. The method of any one of claims 1 to 6, wherein
(a) the first and/or the second plurality of IHC assays comprising at least one blue chromogen;
(b) the first and/or the second plurality of IHC assays comprising at least one brown chromogen; and/or
(c) the first plurality and/or the second plurality of IHC assays comprising at least one red chromogen.

8. The method of any one of claims 1 to 6, the first plurality and/or the second plurality of IHC assays comprising at least one green chromogen.

9. The method of any one of the preceding claims, wherein each IHC assay comprises
(a) contacting the tissue sample with a secondary antibody conjugated to an enzyme configured to catalyze a chromogen, the secondary antibody being specific to the primary antibody; and
(b) detecting the chromogen.

10. The method of any one of the preceding claims, wherein:
A) each of the tissue section comprises tissue obtained from an individual having, or suspected of having, a CUP; and/or
B) each of the tissue section comprises tissue obtained from the same individual; and/or
C) each of the tissue section comprising tissue from the same biopsy sample or each of the tissue section comprises tissue from different biopsy sample; and/or
D) the first tissue section and the second tissue section are the same or the first tissue section and the second tissue section are different; and/or
E) the method further comprises staining the first and/or second tissue section with hematoxylin; and /or
F) the method further comprises imaging the first and/or the second tissue section; and/or
G) the method further comprises detecting a first chromogen, a second chromogen, and a third chromogen, in particular wherein the first chromogen, the second chromogen, and the third chromogen are simultaneously detected.

11. A method for an automated multiplex IHC assay of one or more tissue samples disposed on a slide, the method comprising dispensing one or more reagents to the one or more tissue samples using one or more automated fluid dispensers in a series of staining steps associated with an IHC protocol, the reagents comprising
a first reagent comprising a primary antibody specific to at least one biomarker selected from smooth muscle actin, S100, CD45, CD34, desmin, and cytokeratin;
a second reagent comprising a secondary antibody specific for the first reagent; and
at least one chromogen.

12. The method of claim 11, further comprising dispensing one or more reagents to the one or more tissue sections using one or more automated fluid dispensers in a series of sample preparation steps associated with a sample preparation protocol.

13. The method of claim 11 or 12, wherein:
A) the primary antibody comprises a mouse monoclonal antibody and/or a rabbit monoclonal antibody; and/or
B) the secondary antibody comprising an anti-species antibody; and/or
C) the secondary antibody comprising an anti-mouse and/or anti-rabbit antibody.

14. The method of any one of the preceding claims, wherein the method is run on an automated slide staining apparatus.

15. A kit comprising a plurality of primary antibodies, the plurality of primary antibodies comprising a primary antibody specific to smooth muscle actin, a primary antibody specific to S100, a primary antibody specific to CD45, a primary antibody specific to CD34, a primary antibody specific to desmin, and a primary antibody specific to cytokeratin.
